Europäisches Patentamt

European Patent Office

Office européen des brevets

(11) Numéro de publication: **0 298 807 B1**

# FASCICULE DE BREVET EUROPEEN

(45) Date de publication de fascicule du brevet: **28.12.94**

(51) Int. Cl.5: **C12N 5/16**, C12N 15/12, C12N 15/90, C12P 21/02, A61K 37/64, A61K 37/48

(21) Numéro de dépôt: **88401520.7**

(22) Date de dépôt: **17.06.88**

(54) **Procédé de préparation de lignées cellulaires stables pour la production de protéines déterminées, à partir d'animaux transgéniques; lignées cellulaires tumorales et protéines obtenues.**

(30) Priorité: **19.06.87 FR 8708623**

(43) Date de publication de la demande:
**11.01.89 Bulletin 89/02**

(45) Mention de la délivrance du brevet:
**28.12.94 Bulletin 94/52**

(84) Etats contractants désignés:
**AT BE CH DE ES FR GB GR IT LI LU NL SE**

(56) Documents cités:
**EP-A- 0 169 672**
**FR-A- 2 487 851**

**NATURE, vol. 315, 9 mai 1985, pages 115-122; D. HANAHAN: "Heritable formation of pancreatic beta-cell tumours in transgenic mice expressing recombinant insulin/simian virus 40 oncogenes"**

(73) Titulaire: **TRANSGENE S.A.**
**11, rue de Molsheim**
**F-67000 Strasbourg (FR)**

(72) Inventeur: **Skern, Tim**
**15, rue de St Dié**
**F-67100 Strasbourg (FR)**
Inventeur: **Courtney, Michael**
**11 rue des Lilas**
**F-67170 Geudertheim (FR)**
Inventeur: **Lecocq, Jean-Pierre**
**6, rue du champ du Feu**
**F-67116 Reichstett (FR)**

(74) Mandataire: **Warcoin, Jacques et al**
**Cabinet Régimbeau,**
**26, avenue Kléber**
**F-75116 Paris (FR)**

Rank Xerox (UK) Business Services
(3.10/3.09/3.3.3)

PROCEEDINGS OF THE NATIONAL ACADEMY OF SCIENCES, vol. 84, mars 1987, pages 1299-1303; A.-C. ANDRES et al.: "Ha-ras on-cogene expression directed by a milk protein gene promoter: Tissue specificity, hormonal regulation, and tumor induction in transgenic mice"

NATURE, vol. 318, 12 décembre 1985, pages 533-538; J.M. ADAMS et al.: "The c-myc oncogene driven by immunoglobulin enhancers induces lymphoid malignancy in transgenic mice"

JOURNAL OF CELLULAR BIOCHEMISTRY, vol. 0, no. 12, suppl. 1988, no. H220, page 193; A. PAVIRANI et al.: "Oncogene-induced T cell tumours expressing a foreign protein in transgenic mice"

EP 0 298 807 B1

## Description

L'invention concerne la mise au point de nouvelles lignées cellulaires pour la production de protéines complexes.

Depuis quelques années, un nombre de plus en plus important de protéines a été produit par les techniques de l'ADN recombinant (hormones, lymphokines ...) dans différents types de cellules hôtes, bactéries ou levures. Dans de nombreux cas, ces microorganismes apparaissent comme des moyens de production efficaces et rentables parce que leur génétique et leur physiologie sont bien connues et les techniques de fermentation, à l'échelle industrielle, sont bien maîtrisées.

Cependant, pour des protéines complexes qui ne sont actives qu'après avoir subi des modifications post-traductionnelles, on a constaté que l'expression dans les bactéries et les levures ne permet pas toujours de telles modifications. Dans ces cas là, on n'obtient pas de protéines biologiquement actives. Par exemple, on sait que des molécules d'importance thérapeutique comme les facteurs VIII et IX de la coagulation sanguine doivent être produites dans des cellules de mammifères, et peut être même dans des types cellulaires spécifiques.

De nombreuses études tentent donc de mettre au point des systèmes performants qui permettent l'expression de gènes étrangers clonés dans des cellules eucaryotes supérieures.

Les différents travaux réalisés se sont heurtés à un certain nombre de difficultés telles qu'un rendement pas toujours satisfaisant, un faible nombre de systèmes performants, l'instabilité des gènes étrangers transfectés, ou encore la nécessité, pour la production de protéines telles que le facteur IX qui doivent subir des modifications post-traductionnelles complexes, de choisir un type bien spécifique de cellules.

La présente invention vise à mettre au point de nouvelles approches expérimentales pour la production de protéines recombinantes complexes par les lignées cellulaires qui, au moins partiellement, ne présentent pas les inconvénients mentionnés ci-dessus.

Pour atteindre son but, l'invention propose d'utiliser les techniques permettant la création d'animaux transgéniques.

Les techniques qui permettent la création de souris transgéniques sont bien décrites (voir revue de Palmiter et Brinster, 1986). En résumé, une préparation d'ADN comportant un gène étranger entouré des signaux de contrôle appropriés pour permettre son expression dans les cellules supérieures, est microinjectée in vitro dans le pronucleus d'un oeuf fertilisé.

Les oeufs injectés sont transplantés dans des femelles porteuses. Parmi les souriceaux qui parviennent à terme, le gène étranger ou "transgène" est intégré dans le matériel chromosomique à une fréquence de 10 à 15 %. Dans la majorité des cas, le gène est intégré dans les cellules germinales et devient ainsi transmissible aux générations suivantes de souriceaux.

Des techniques semblables ont également été décrites pour d'autres espèces animales (porc, mouton, vache).

On constate généralement que les souris transgéniques expriment le "transgène" spécifiquement dans certains tissus qui reproduisent l'environnement naturel où le gène est exprimé, dans son hôte d'origine. Par exemple, les souris transgéniques porteuses du gène de l'élastase du rat synthétisent de l'élastase de rat dans leur pancreas (Swift et al. 1984) ; le gène de l'$\alpha$-antitrypsine humaine est exprimé à un haut niveau dans le foie des souris (Sifers et al. 1987). Cette spécificité tissulaire est souvent déterminée par les séquences du promoteur (ou proches de celui-ci) du gène étranger. Ceci est démontré par l'étude de fusions entre des gènes et des promoteurs hétérologues. Par exemple la fusion du gène CAT (chloramphénicol acétyltransférase) de bactérie avec le promoteur du gène de la protéine $\alpha$A du cristallin de souris, entraîne la synthèse de CAT dans le cristallin des souris transgéniques et une régulation de son expression au cours du développement de la souris (Overbeek et al. 1985).

Il est donc possible de diriger l'expression des gènes vers certains tissus ou catégories de tissus, dans les animaux transgéniques, en utilisant des séquences promoteur appropriées. Plusieurs travaux décrivent l'expression d'oncogènes cellulaires ou viraux dans les souris transgéniques, par exemple c-myc, c-ras, c-fos et l'antigène T du SV40 (Adams et al. 1985 ; Quaife et al. 1987 ; Rütner et al. 1987 ; Palmiter et al. 1987).

L'expression de ces oncogènes peut aboutir au développement de tumeurs dans les tissus qui l'expriment. Par exemple, des constructions d'ADN qui comportent l'oncogène c-myc et une séquence "enhancer" proximale de la chaîne lourde des immunoglobulines (Ig) induisent, dans les souris transgéniques, des tumeurs lymphoïdes les lignées cellulaires B, dont la spécificité est due à l'effet du "enhancer" de l'Ig (Adams et al. 1985).

Ces expériences montrent donc qu'il est possible de provoquer l'apparition d'une tumeur dans un tissu ou dans un type de cellule choisi, en utilisant un oncogène approprié et un promoteur et/ou un "enhancer"

3

EP 0 298 807 B1

spécifique d'un tissu.

Les tumeurs induites artificiellement par ces consstructions d'ADN peuvent être mises en culture in vitro et on peut en dériver des lignées cellulaires établies. Adams et al. (1986) ont décrit la sélection de lignées permanentes de cellules B à partir de tumeurs induites par l'expression constitutive de c-myc.

La présente invention vise à fournir un nouveau procédé permettant la préparation d'une protéine déterminée, ce procédé utilisant la technique de création d'animaux transgéniques.

Plus précisément, l'invention a pour objet un procédé de préparation d'une lignée cellulaire stable produisant une protéine déterminée, caractérisé en ce que :

a) - on prépare un vecteur comportant

- une séquence oncogène ainsi que les éléments assurant son expression dans une cellule eucaryote supérieure,
- un bloc d'expression qui comprend au moins la séquence codant pour ladite protéine ainsi que les éléments assurant son expression dans ladite cellule eucaryote supérieure tumorale ; ladite protéine étant différente de celle exprimée par ladite sèquence oncogène,

b) - on introduit ce vecteur dans un ou plusieurs oeufs d'un animal et on réimplante le ou les oeufs obtenus ;

c) - on sélectionne les descendants ayant intégré l'oncogène et qui développent des tumeurs ;

d) - on prélève et on cultive sur un milieu approprié les cellules tumorales ainsi obtenues et qui produisent la protéine déterminée afin d'obtenir une lignée cellulaire.

Sous un des aspects de l'invention, on peut, avant leur mise en culture, réimplanter les cellules tumorales plusieurs fois successivement dans d'autres animaux.

Dans une variante du procédé selon l'invention, on peut produire la protéine déterminée de la façon suivante :

a) - on prépare un vecteur comportant

- un bloc d'expression qui comprend au moins la séquence codant pour ladite protéine ainsi que les éléments assurant son expression dans une cellule eucaryote supérieure ;

b) - on introduit ce vecteur dans un ou plusieurs oeufs d'un animal et on réimplante le ou les oeufs obtenus ;

c) - on sélectionne les descendants ayant intégré la séquence codant pour ladite protéine ;

d) - on fusionne les lymphocytes de la rate de ces animaux avec des cellules de myelome et on sélectionne des clones d'hybridome produisant la protéine.

L'invention propose en particulier des moyens pour donner une spécificité tissulaire à l'expression de l'oncogène, de la protéine déterminée, ou des deux.

Il s'agit par exemple d'utiliser des signaux de contrôle puissants de l'expression dans les cellules lymphoïdes pour optimiser la production de protéines étrangères dans les transhybridomes.

L'induction spécifique de tumeurs dans les souris transgéniques peut être déclenchée par différents oncogènes et ceux-ci peuvent être placés sous le contrôle de différents promoteurs pour influencer la spécificité tissulaire de la réponse.

On appellera "oncogène" dans la présente description le gène ayant la capacité d'induire chez l'animal une prolifération cellulaire du type de celle qui conduit à la formation de tumeur.

Parmi les oncogènes utilisables, il faut citer plus particulièrement les oncogènes d'origine virale quelles que soient leurs origines. Ainsi, il faut citer les oncogènes tels que c-myc, c-ras, c-fos ou l'antigène T de SV40, mais d'autres oncogènes peuvent être envisagés tels que les oncogènes src, erb, myb et onc par exemple.

De façon générale, le vecteur comportera à titre d'éléments d'expression tant de l'oncogène que de la séquence codant pour la protéine, un promoteur et également un activateur de transcription ("enhancer").

Par exemple, le promoteur et l'activateur de transcription ("enhancer") de la chaîne lourde des Ig peuvent être utilisés pour diriger l'expression vers les cellules lymphoïdes ; l'introduction de c-myc ou de l'antigène T de SV40 dans la même construction, induira le développement de lymphomes invasifs.

Selon le même principe, pour induire des tumeurs du foie on utilisera des promoteurs spécifiques des cellules hépatiques pour diriger l'expression de l'oncogène. On utilisera par exemple le promoteur de l'α-antitrypsine, du facteur VIII ou du facteur IX ou de l'albumine.

Théoriquement, on peut induire une tumeur de n'importe quel type de cellule pour autant que l'on associe le promoteur, le "enhancer" et l'oncogène appropriés.

Plus particulièrement, pour engendrer des tumeurs produisant une protéine étrangère, ce qui constitue l'objet de la présente invention, on placera le gène étranger sous le contrôle d'un promoteur efficace dans les cellules tumorales.

4

Ainsi dans le cas des cellules lymphoïdes on a constaté que les éléments régulateurs de l'immunoglobuline étaient particulièrement efficaces.

Plus particulièrement, dans le cas des cellules de lymphome induites par c-myc sous le contrôle du promoteur et du "enhancer" de la chaîne lourde des Ig, les gènes étrangers peuvent être placés sous le contrôle du promoteur de la chaîne légère des Ig, et également sous l'influence du même élément "enhancer" de la chaîne lourde. (On peut indifféremment inverser les 2 promoteurs et placer le gène étranger sous le contrôle du promoteur de la chaîne lourde et l'oncogène sous le promoteur de la chaîne légère).

Ce type de construction aboutit à une sécrétion efficace de la protéine étrangère par la cellule tumorale, aussi bien in vivo (dans ce cas la protéine se retrouve dans la circulation sanguine de la souris transgénique) et in vitro après mise en culture de lignées cellulaires dérivées des tumeurs.

De la même manière, dans le cas des cellules de tumeur du foie, les gènes étrangers peuvent être placés sous le contrôle de promoteurs puissants dans les cellules hépatiques, comme le promoteur de l'$\alpha$-antitrypsine, de l'albumine, éventuellement additionnés de l'élément "enhancer" du virus de l'hépatite B (Babinet et al. 1985).

Il est encore possible d'utiliser des promoteurs moins spécifiques, de manière à engendrer des tumeurs de plusieurs types (Palmiter 1986). Parmi les éléments couramment utilisés on peut citer le promoteur-"enhancer" de SV40, le promoteur majeur tardif de l'adénovirus, le promoteur de la métallothionéine de souris et le promoteur H-2 de souris.

Les éléments promoteurs des Ig peuvent encore être utilisés pour augmenter l'expression de gènes étrangers dans les hybridomes issus de la fusion de cellules de rate de souris transgéniques avec des cellules de myelome.

L'isolement de transhybridomes exprimant l'hormone de croissance humaine a été décrit (Babinet et al. 1986) ; le gène est placé sous le contrôle du promoteur H-2K de souris, qui est un promoteur assez faible mais avec un large spectre de cellules hôtes.

Les techniques permettant l'introduction du vecteur dans les oeufs sont connues ; en particulier, s'il est utile, afin de construire le vecteur, d'utiliser des plasmides comme intermédiaire de synthèse, il est par contre préférable d'introduire le vecteur sous forme d'ADN linéaire et débarrassé autant que faire se peut des séquences bactériennes.

Les techniques de réimplantation, de sélection et de culture sont elles aussi connues et certaines seront décrites plus en détail dans les exemples ci-annexés.

On peut encore augmenter le niveau d'expression des gènes étrangers dans les cellules tumorales ou dans les transhybridomes en liant, à ces gènes, un gène amplifiable de résistance à un inhibiteur (par exemple les gènes de la dihydrofolate réductase ou de l'adénine déaminase). Après traitement des cellules, in vitro, avec l'inhibiteur correspondant (méthotréxate ou 2′-déoxycoformycin) on peut sélectionner des clones qui présentent une co-amplification du gène à exprimer et du marqueur de sélection et donc une augmentation de l'expression du gène étranger.

La présente invention permet le développement de nouveaux systèmes de production des protéines d'intérêt thérapeutique.

. L'inhibiteur de l'élastase neutrophile humaine, l'$\alpha$-antitrypsine et ses variants du site actif $\alpha$AT (Leu$^{358}$), $\alpha$AT (Arg$^{358}$) et $\alpha$AT (Ala$^{357}$, Arg$^{358}$), destinés au traitement de l'emphysème pulmonaire et de troubles thrombotiques et d'hypertension, peuvent être produits par de nouvelles lignées cellulaires, selon l'invention.

. De nouvelles lignées cellulaires dérivées de tumeurs induites dans le foie permettant la production de protéines qui nécessitent un important "processing" post-traductionnel, comme le facteur IX, la protéine C et le facteur VIII de la coagulation sanguine, l'activateur tissulaire du plasminogène (TPA), la pro-urokinase, etc., ainsi que des antigènes viraux (par exemple divers antigènes du virus HIV).

. D'une manière plus générale, la présente invention peut être utilisée pour l'expression et la production de toutes les protéines qui doivent être synthétisées dans des cellules eucaryotes supérieures.

. La technique n'est pas limitée à l'espèce souris mais peut également être appliquée au rat, au poulet, au lapin, au porc, à la vache, aux batraciens et aux poissons.

L'invention concerne également les animaux transgéniques obtenus au cours de la mise en oeuvre du procédé selon l'invention, ainsi que les cellules et les lignées cellulaires obtenues.

Enfin, l'invention a pour objet les protéines obtenues par le procédé selon l'invention, et plus particulièrement des protéines d'origine hépatique telles que l'alpha-antitrypsine et ses variants, ou les facteurs VIII et IX de la coagulation sanguine et leurs analogues ayant sensiblement la même activité thérapeutique.

D'autres caractéristiques et avantages de l'invention apparaîtront au cours de la description suivante illustrée par les figures 1 à 11 :

Figure 1 : Structure du gène de la chaîne légère K d'immunoglobuline de souris, dérivé de la lignée de myélome MPC11.

Figure 2 : Structure du pTG1999
HCE = "heavy chain enhancer"
SS = séquence signal

Figure 3 : Construction du vecteur pTG1993 comportant les éléments de contrôle de transcription et de traduction de l'immunoglobuline.
Abréviations : HCE "heavy chain enhancer" ; $V_{LR}$ promoteur de la chaîne légère ; sites de restriction :

| B | BamHI | E | EcoRI |
| P | PstI | H | HpaI |
| St | StuI | X | XhoI |
| N | NsiI | Ha | HpaI |
| Hc | HincII | C | ClaI |
| | | Sm | SmaI |

Figure 4 : Introduction de l'oligonucléotide TG616/617 dans le site NsiI du pTG1958.

Figure 5 : Création d'un site ClaI dans l'intron portant la séquence signal de la chaîne légère Ig, dans le M13TG1930.

Figure 6 : Construction du plasmide pTG1990 permettant l'introduction du cADN de l'$\alpha_1$AT dans un plasmide portant les éléments de contrôle de transcription et traduction de l'immunoglobuline (abréviations : voir figure 3 - M = méthionine ; A = arginine).

Figure 7 : Introduction d'un site SmaI dans la région 3′ non traduite du cADN de l'$\alpha_1$AT (mêmes abréviations que dans la figure 3).

Figure 8 : Construction du pTG1999 à partir des plasmides pTG1993 et pTG1990.

Figure 9 : Détail de la structure du pTG1999 à partir du "Start" de l'exon 2).

Figure 10 : Introduction du gène du grand antigène T du SV40 sous le contrôle du promoteur de la chaîne lourde d'Ig dans le pTG1999 (mêmes abréviations que dans la figure 3).

Figure 11 : Introduction du gène c-myc sous le contrôle du promoteur de la chaîne lourde Ig dans le pTG1999 (mêmes abréviations que dans la figure 3).

Méthodes

1) Manipulations de l'ADN

D'une manière générale, on utilisera les méthodes classiques, décrites dans "Molecular cloning, a laboratory manual" (1982) R. Maniatis, E.F. Fritsch et J.B. Sambrook, Cold Spring Harbor press.

a. Construction de nouvelles molécules d'ADN

a.1. Préparation des vecteurs et des fragments

Les enzymes de restriction sont utilisés conformément aux instructions du fournisseur (New England Biolabs et Promega Biotech).

On incube 1-2 $\mu$g d'ADN dans 100 $\mu$l avec la quantité d'enzyme nécessaire pour donner une digestion complète en 2 heures à 37°C. (La digestion est vérifiée par analyse électrophorétique en gel d'agarose) Pour diminuer le bruit de fond et empêcher la religation des vecteurs, ils sont traités à la phosphatase alcaline bactérienne ou à la phosphatase intestinale de veau, ce qui élimine les extrémités 5′ phosphates (on utilise 20 unités de phosphatase en tampon Tris 0,1 M pH 8,0, pendant 2 heures à 37°C). L'ADN est purifié par extraction au phénol-chloroforme puis précipité à l'éthanol et redissous en tampon Tris 10 mM pH7,5 EDTA 1 mM à une concentration d'environ 100 ng/$\mu$l.

Les vecteurs à bouts francs sont préparés par digestion à la nucléase $S_1$ de Aspergillus oryzae. On incube 5 $\mu$g d'ADN, clivé par un enzyme de restriction, avec 10 unités de nucléase $S_1$ dans un volume de 100 $\mu$l de solution de NaCl 0,3 M, $CH_3COONa$ 0,03 M pH 4,8 et $ZnCl_2$ 0,003 M. La réaction est poursuivie à 37°C pendant 20 minutes puis elle est arrêtée par addition d'EDTA 10 mM. L'ADN est purifié comme décrit plus haut.

Les fragments d'ADN à insérer dans les vecteurs sont préparés de la manière suivante : 5 à 10 $\mu$g d'ADN sont digérés par les enzymes de restriction appropriés et les fragments sont séparés par électrophorèse sur gel d'agarose puis le fragment choisi est récupéré à partir du gel (selon la méthode classique de Smith H.O. Methods in Enzymology (1980) 65, 371-380, ED. Grossman et Moldave).

Les fragments obtenus après mutagénèse dans les vecteurs M13 ne sont pas repurifiés avant insertion dans le vecteur puis-qu'il n'y a pas de bruit de fond dû au M13 après sélection en ampicilline.

Les oligonucléotides synthétiques sont traités à la kinase avant d'être utilisés (on incube pendant 30 minutes à 37°C, 200 ng d'oligonucléotide 20 $\mu$l de mélange réactionnel : Tris 50 mM pH 7,6, $MgCl_2$ 10 mM, 2-mercaptoéthanol 10 mM, spermidine 0.1 mM, EDTA 0,1 mM et 10 unités de $T_4$ polynucléotide kinase).

Les oligonucléotides destinés à des mutagénèses dirigées sont récupérés à ce stade, par précipitation à l'éthanol en présence de 10 $\mu$g de t-ARN porteur.

Les oligonucléotides servant de "linkers" ou d'adaptateurs sont traités de la manière suivante :
- quand les 2 brins ne sont pas identiques, ils sont soumis à la kination ensemble ; l'appariement des 2 brins (ainsi que l'appariement des brins complémentaires) se fait par chauffage à 65°C pendant 10 minutes suivi d'un refroidissement lent à 30°C puis à 4°C pendant au moins 4 heures.

a.2. Mutagénèse dirigée de fragments d'ADN

Les fragments d'ADN à muter sont insérés dans un des dérivés simple brin du phage M13 et sont appariés avec des oligonucléotides de synthèse portant la séquence voulue.

a.3. Ligation des fragments d'ADN

Les mélanges de ligation comprennent généralement 100 ng d'ADN du vecteur et 50 à 200 ng d'ADN du fragment à insérer ou 100 à 200 p.moles d'oligonucléotide, dans 20 $\mu$l de tampon Tris 10 mM pH 7.5, $MgCl_2$ 6mM, 2-mercaptoéthanol 6 mM et ATP 2 mM. L'incubation se fait à 14°C pendant 16 heures ou à température ambiante pendant 2 heures.

a.4. Transformation des cellules compétentes

Des cellules compétentes des souches de E. coli 5 K (pour les plasmides) et JM103 (pour les M13) sont transformées selon les méthodes classiques.

b. Vérification des constructions

Des préparations d'ADN à petite échelle sont réalisées à partir des colonies résistantes à l'ampicilline ou à partir des plages de M13 et l'ADN est analysé par enzymes de restriction ou par séquençage.

Le séquençage de l'ADN est effectué par la méthode de Sanger et al. (Proc. Natl Acad. Sci. 1977, 74, 5463).

c. Préparation des fragments d'ADN en gradient de sucrose

Les gradients de sucrose (15-25 %) sont préparés en tampon Tris 50 mM pH7,5 EDTA 1 mM ; l'ADN clivé, à 10 $\mu$g dans un volume maximum de 300 $\mu$l est chargé au sommet du gradient et mis à centrifuger à 20°C pendant 16 heures à 30 K.

Des fractions successives de 400 $\mu$l sont récoltées par le sommet du gradient et analysées par électrophorèse sur gel d'agarose d'un aliquot de 15 $\mu$l. Dans les fractions choisies, l'ADN est précipité à l'éthanol et purifié par extraction au phénol-chloroforme, précipité à l'éthanol et redissous en tampon TE. La pureté de la préparation est confirmée par électrophorèse sur gel. Les dilutions approrpiées sont réalisées pour l'injection (de l'ordre de 5-10 ng/ml).

2) Technique d'obtention de souris transgéniques par injection d'ADN dans l'oeuf de la souris

On utilise :
- Des souris capables de produire beaucoup d'ovules et prêtes à être fécondées par des souris mâles. On choisira pour cela des souris de la souche C57xSJL que l'on traitera avec des hormones sexuelles.
- Des souris capables de supporter une gestation ; pour cela on utilisera des souris blanches "Swiss" qui ont déjà subi une gestation complète.

On utilisera les souris fécondées C57xSJL pour extraire de leurs ovaires les ovules fécondés.

Dans les noyaux des ovules fécondées appelés "oeufs" on injecte l'ADN étranger, puis on réimplante les oeufs dans les ovaires des souris porteuses "Swiss" où ils se développeront.

a) Préparation des souris C57xSJL

Deux hormones sont injectées dans la souris avant que celle-ci ne soit mise avec un mâle.
- 48 heures avant, les souris reçoivent une injection de PMSG (pregnant marc serum gonadotrophine (FSH) à 10 U/souris) à une dose de 0,2 ml par souris. Ce traitement permet d'obtenir des souris qui produisent nettement plus d'ovules que des souris normales.
- Juste avant de mettre les souris femelles avec les mâles on leur injecte 0,2 ml de HCG (Human chorionique gonadotrophine (LH) à 5 U/souris) ceci pour que la femelle accepte l'accouplement avec le mâle.

Les souris restent alors une nuit avec les mâles avec lesquels elles vont s'accoupler (90 % de femelles sont fécondées).

Le lendemain matin elles sont séparées des mâles puis tuées. On extrait les deux ovaires de chaque souris (de 6 à 10 souris par jour) et de chaque ovaire on extrait les ovules et oeufs qui sont contenus dans une poche visible sous microscope.

b) Préparation des oeufs pour l'injection

Pour maintenir les oeufs en bon état, l'ovaire, une fois extrait est mis dans une solution de Brinster dont on équilibre le pH en le laissant dans une étuve à 37°C sous atmosphère de 5 à 6 % de $CO_2$.

Les oeufs extraits de l'ovaire sont encore agglutinés par un tissu folliculeux qu'il faut éliminer en les laissant 20 minutes dans une solution de hyaluronidase. Ainsi individualisés les oeufs sont lavés deux fois dans la solution de Brinstère. Ils sont ensuite sélectionnés car il y a beaucoup d'oeufs aberrants ou crevés. On obtient en général 50 à 60 oeufs. Pour les manipuler ensuite sous le microscope, les oeufs sont déposés dans une goutte de solution Brinstère-Hépès additionnée de cytochalasine. Cette solution permet à l'oeuf d'avoir une meilleure résistance à l'injection.

c) Injection d'ADN dans les oeufs

Par observation au microscope (grossissements x100 et x200) les oeufs sont à nouveau sélectionnés. On sépare ceux qui sont fécondés (ils contiennent deux noyaux) de ceux qui ne le sont pas. Les oeufs fécondés vont alors être injectés.

Pour maintenir l'oeuf en place lors de l'injection celui-ci est aspiré par une grosse aiguille aux bouts arrondis ; l'ADN est injecté avec une aiguille très fine qu'on pique dans un des noyaux ; on poursuit l'injection jusqu'à ce qu'on voie la membrane de ce noyau (nucléoplasme) se dilater.

Les aiguilles sont actionnées à l'aide de micromanipulateurs installés de part et d'autre du microscope. Certains oeufs trop injectés éclatent. On les élimine et on ne garde que les oeufs correctement injectés pour la réimplantation.

d) Préparation des souris blanches "Swiss"

La veille de la manipulation, une quinzaine de souris "Swiss" sont mises avec des males vasectomisés, donc incapables de féconder les ovules de la femelle. Après une nuit avec le mâle il s'agit de repérer quelles femelles se sont accouplées avec les mâles. Pour ce faire il suffit de repérer à l'ouverture de l'utérus de la souris un bouchon translucide formé par le liquide spermatique du mâle. Chaque femelle ayant ce bouchon est donc considérée comme apte à commencer une gestation.

Sur une quinzaine de souris femelles, il y en a environ 1 à 5 qui seront sélectionnées pour la réimplantation.

e) Réimplantation des oeufs dans les souris porteuses

Après l'anesthésie d'une souris "Swiss" on fait une entaille dans la peau de celle-ci, au niveau où se trouve l'ovaire. Celui-ci une fois repéré est retiré hors du corps de la souris. On y repère la poche contenant les oeufs, on y fait une ouverture pour permettre l'introduction de la pipette capillaire dans laquelle on a aspiré les oeufs. On souffle le contenu de la pipette dans la poche et ainsi les oeufs fécondés et injectés sont prêts à partir pour le cycle de la gestation.

On remet ensuite l'ovaire en place et on referme les entailles faites sur la souris.

3) Production d'hybridomes

La fusion des cellules de rate et des cellules de myelome pour produire des hybridomes se fait selon la technique décrite par Köhler, G et Milstein, 1974, Nature 256, 495.

4) Mesure de l'$\alpha_1$-antitrypsine dans le plasma des souris par test ELISA

On utilise un test ELISA "en sandwich", très sensible (capable de détecteur 0,1 ng d'$\alpha_1$-AT) mis au point par Michalski et al. 1985, J. Immunol. Meth. 83, 101-112.

En résumé, un anticorps anti-$\alpha$AT de chèvre est adsorbé sur les cupules des plaques d'immunodétection (Nunc-Immunoplate).

Des dilutions en série d'une solution standard d'$\alpha$-AT et des plasmas à analyser sont réalisées et mises en contact avec les cupules, pendant 1 heure à 37°C. Après rinçage, l'$\alpha$-AT fixée est révélée par un deuxième anticorps, de lapin. Celui-ci va fixer un anticorps anti-lapin, marqué à la biotine, qui va fixer un complexe peroxydase-streptavidine (Amersham). Le complexe fixé sera révélé avec une solution de 0,1 % 0-phenylenediamine (Sigma)-1 % méthanol-0,1 % peroxyde d'hydrogène-$H_2O$. La réaction est arrêtée après 20 minutes par addition d'acide sulphurique 3 M. La réaction colorée est mesurée au spectrophotomètre Titertek-uniscan.

Les valeurs mesurées sont portées sur un graphique à double échelle logarithmique et la courbe établie avec la solution standard d'$\alpha$-AT permet de calculer les concentrations de chaque échantillon.

Exemple 1 Expression d'une protéine étrangère dans des souris transgéniques, en utilisant des éléments régulateurs de transcription et de traduction des immunoglobulines.

A. Construction d'un vecteur d'expression pour l'$\alpha_1$-antitrypsine humaine (variant $Arg^{358}$) : pTG1999

Le gène de l'$\alpha$-antitrypsine humaine a été choisi comme premier modèle. La construction de départ est un plasmide qui porte la séquence codante du variant $Arg^{358}$ de l'$\alpha$-AT, pTG1951 (Ce plasmide ne se distingue du pTG152 (décrit dans le brevet français 86.09608) que par un site de restriction ; tout autre plasmide portant le même bloc d'expression pourrait être utilisé).

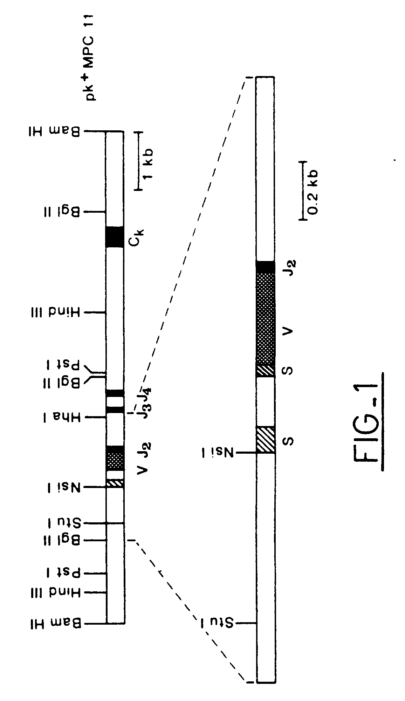

La séquence codante de l'$\alpha$-AT va être placée sous le contrôle des éléments régulateurs de l'immunoglobuline de souris provenant du gène réarrangé de la chaîne légère K, MPCII, décrit par Kelley et al. (1982) (voir figure 1).

La protéine de la chaîne légère est codée par 3 exons : l'exon 1 code pour la plus grande partie de la séquence signal (responsable de l'excrétion de la protéine hors des cellules), l'exon 2 code pour les 4 derniers acides aminés de la séquence signal et pour la région variable et l'exon 3 code pour la région constante. Le promoteur est situé dans les 200 paires de bases qui précèdent le premier exon et le "enhancer" de transcription est situé dans le second intron. Il est encore possible que d'autres régions soient importantes pour la transcription et la stabilité du RNAm.

Pour fusionner la séquence codante de l'$\alpha$-AT à la séquence signal de la chaîne légère, on est amené à déléter toutes les séquences en 3' de la séquence signal y compris le "enhancer". Cette délétion peut être compensée par l'insertion, en amont du promoteur, du "enhancer" de la chaîne lourde des immunoglobulines.

D'autre part, on ajoute en aval de la séquence $\alpha$-AT le site de polyadénylation du SV40.

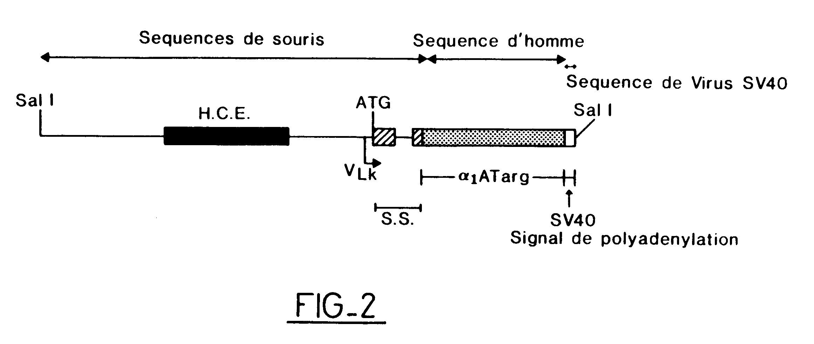

La structure du plasmide pTG1999 est schématisée dans la figure 2.

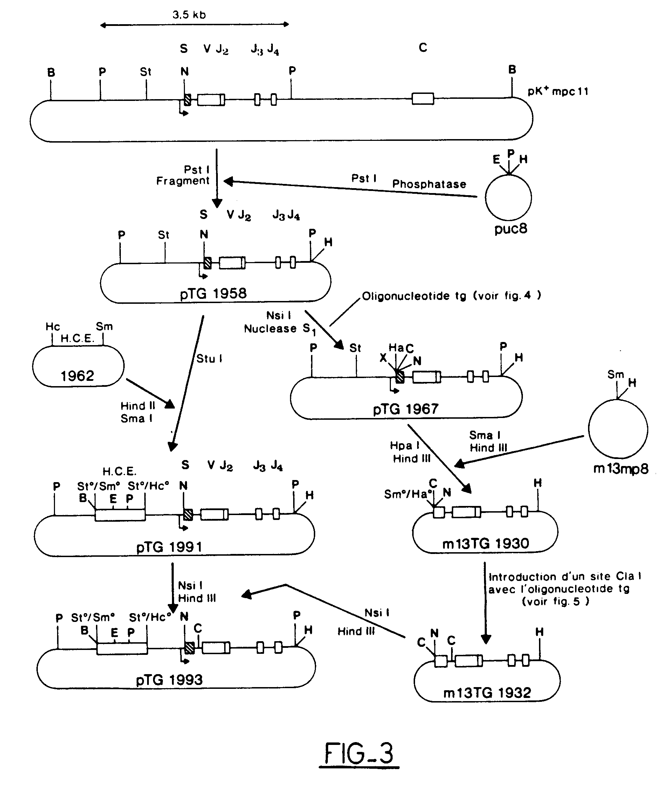

La manipulation des séquences de la chaîne légère de l'Ig est schématisée dans la figure 3 : le fragment PstI de 3,5 kb du pK$^+$ MPC11 qui contient la séquence signal et la séquence codant pour la région variable a été sous-cloné dans le site PstI du plasmide pUC8 pour donner le pTG1958.

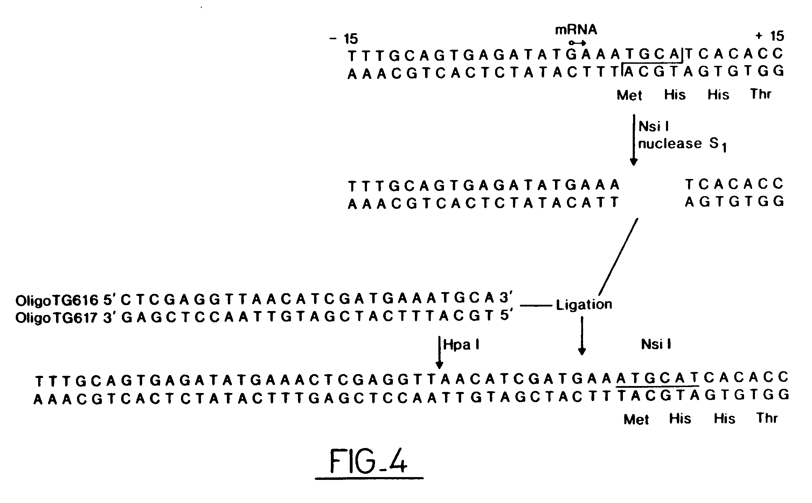

Ce plasmide a été ouvert au site unique NsiI et les extrémités ont été rendues franches par digestion à la nucléase S1, ce qui donne le pTG1967. Un petit oligonucléotide (TG616/617, représenté à la figure 4) a été introduit dans le site NsiI ; cet oligonucléotide comprend les sites de reconnaissance par les enzymes de restriction : XhoI, HpaI, ClaI.

Après insertion de l'oligonucléotide dans l'orientation voulue, le site NsiI est régénéré d'un côté. Cette manipulation permet la récupération du fragment NsiI-HindIII du pTG1958 sous forme de fragment HpaI-HindIII pour l'introduire dans un vecteur M13, pour permettre diverses manipulations et le réintroduire dans pTG1958 ou un de ses dérivés, sous forme de fragment NsiI-HindIII. Parmi plusieurs clones qui ont intégré l'oligonucléotide, on retient le pTG1967 dont le séquençage montre qu'il en a intégré une seule copie et dans l'orientation correcte (figure 4).

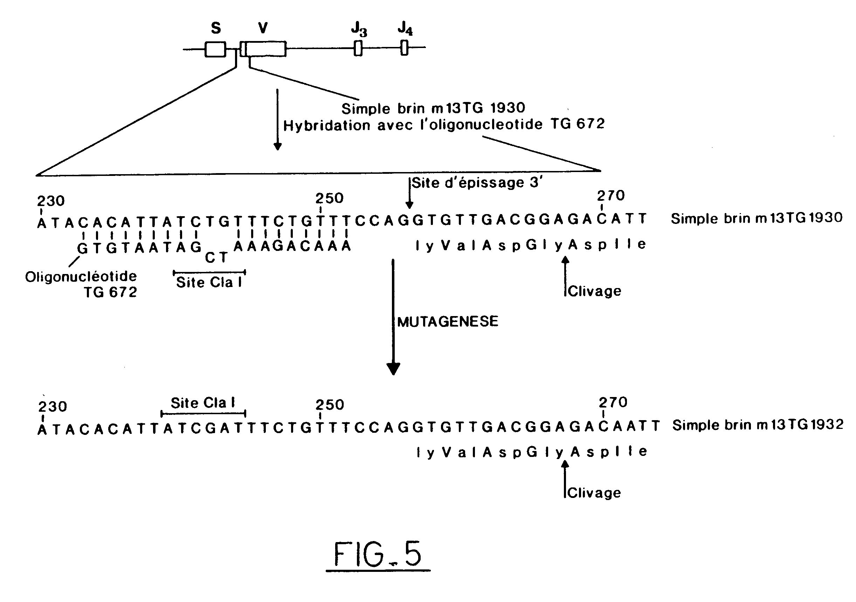

Le fragment HpaI-HindIII de 2 kb est ensuite introduit dans le M13mp8 ouvert par SmaI et HindIII, pour donner le M13TG1930. Par mutagénèse dirigée, on crée un site unique ClaI à la jonction entre la séquence signal et la séquence codante de la chaîne légère, pour permettre l'introduction d'un gène étranger. Le site ClaI est créé par le changement de 2 nucléotides (voir figure 5) à l'intérieur de l'intron ce qui ne doit pas entraîner de conséquence au niveau de la séquence en acides aminés ; de plus cette mutation est suffisamment éloignée du site accepteur d'"épissage" normal. La figure 5 détaille la stratégie qui a été suivie.

Un clone a été sélectionné : M13TG1932.

Le fragment NsiI-HindIII de 2 kb du M13TG1932 a été récupéré et réintroduit dans le pTG1991, un dérivé de pTG1958.

Le pTG1991 est un dérivé du pTG1958 dans lequel on a introduit un fragment de 0.9 kb contenant le "enhancer" de la chaîne lourde de l'immunoglobuline, dans le site unique StuI, environ 0,6 kb en amont de l'ATG initiateur de la chaîne légère.

Le fragment contenant le "enhancer" de la chaîne lourde de l'Ig est le fragment XbaI-XbaI décrit par Church et al. (1985).

Ce fragment a été cloné dans le site XbaI de pUC18 pour donner le pTG1962 ; il a ensuite été récupéré sous forme d'un fragment SmaI-HindIII pour être introduit dans le site StuI du pTG1958. Un clone a été retenu, pTG1991, qui présente le "enhancer" dans l'orientation voulue, comme montré dans la figure 3.

Le pTG1991 a été ouvert par NsiI et HindIII et traité à la phosphatase, pour permettre l'insertion du fragment NsiI-HindIII du M13TG1932 (décrit plus haut), non phosphaté. Après ligation et transformation, les recombinants sont analysés et sélectionnés pour la présence du site ClaI. Un des candidats a été retenu : pTG1993. Ce plasmide est prêt à recevoir l'insertion d'un gène étranger à exprimer, en particulier, le gène de l'$\alpha_1$-antitrypsine.

Le plasmide pTG1951 (brevet français 86.09608) porte un insert BglII-PstI de 1,3 kb qui contient le cADN de la séquence signal et de la protéine mature de l'$\alpha_1$-antitrypsine humaine, variant Arg.

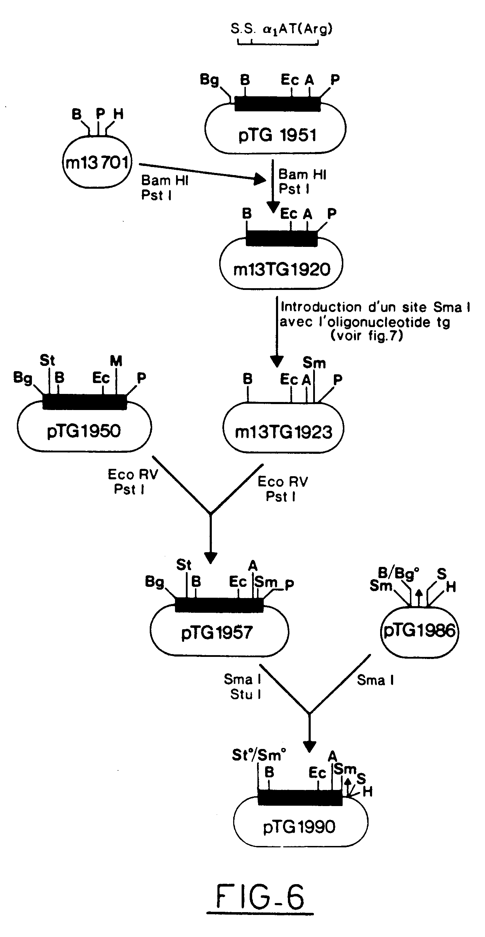

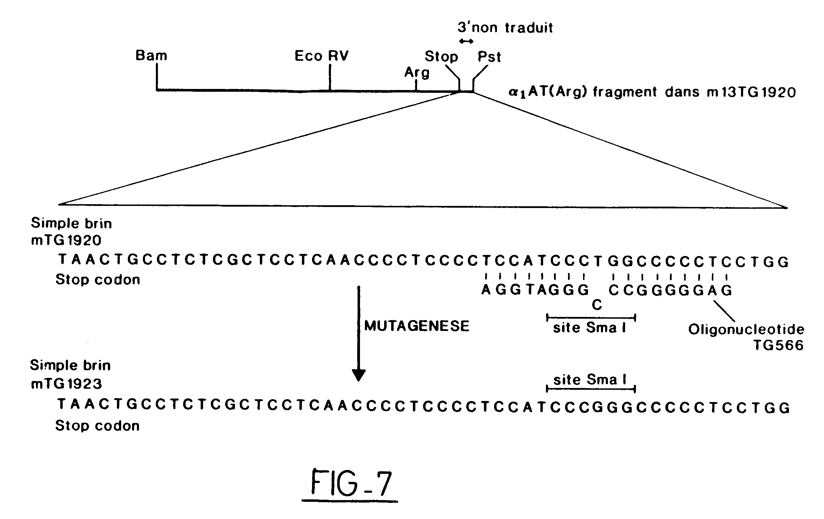

Pour introduire le signal de polyadénylation du SV40 en aval du codon stop du gène $\alpha$-AT, il est nécessaire de créer un site de clivage par un enzyme de restriction. Pour cela, on effectue une mutagénèse dirigée, dans un vecteur M13 (la stratégie est schématisée dans la figure 7) : le fragment BamHI-PstI de 1.1 kb du pTG1951 est introduit entre les sites BamHI et PstI du M13mp701, pour donner le M13TG1920. Avec un nucléotide de synthèse, on crée un site SmaI, à l'extrémité 3′ du M13TG1920. On retient un candidat, M13TG1923, dont la structure est vérifiée par séquençage.

Un fragment EcoRV-PstI de 0,45 kb est récupéré à partir de cette construction et réintroduit entre les sites EcoRV et PstI du pTG1950 pour donner le pTG1957 (voir figure 3).

Le signal de polyadénylation de SV40 peut être récupéré à partir d'un plasmide pTG1986 qui dérive du pUC8 par l'addition d'un fragment SalI-BglII de 0.12 kb (ce fragment contient les nucléotides 2533 à 2666 du virus SV40) qui porte le site de polyadénylation du SV40 orienté de telle sorte qu'il est actif dans le sens 5′→3′ par rapport au site SmaI.

On récupère un fragment StuI-SmaI de 1,1 kb du pTG1957 pour l'introduire dans le site SmaI du pTG1986 de telle manière que le site BamHI de l'insert soit distal par rapport au site polyA. Cette construction est appelée pTG1990.

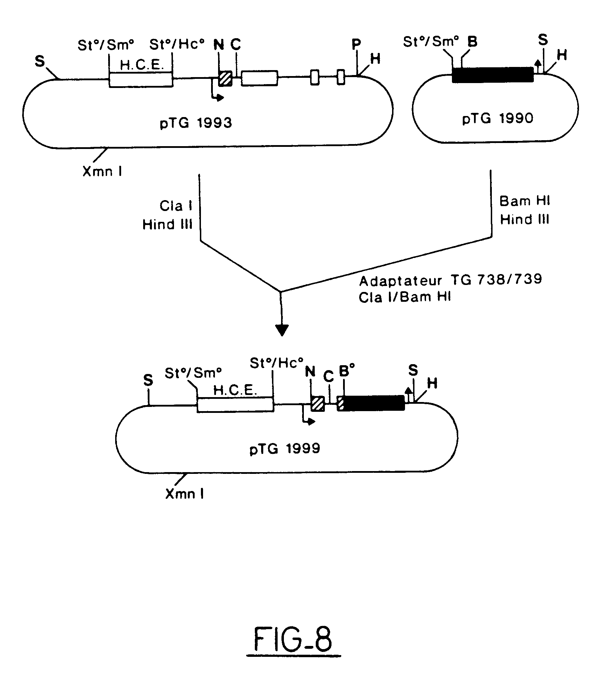

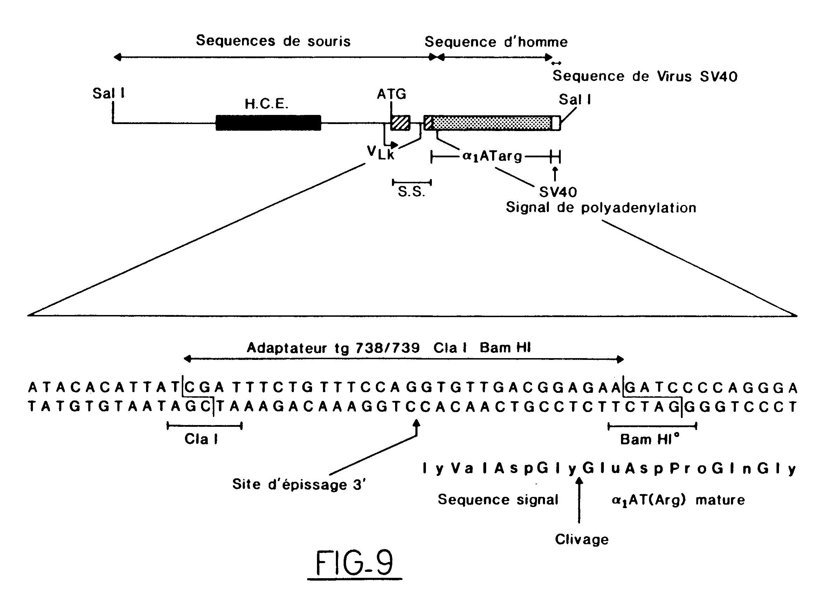

La dernière étape de la construction est le remplacement du fragment ClaI-HindIII du pTG1993 par le fragment BamHI-HindIII du pTG1990. Pour relier une extrémité ClaI avec BamHI, on utilise un adaptateur de synthèse (voir figures 8 et 9) ; celui-ci comprend également les 2 premiers codons correspondant à l'$\alpha_1$-antitrypsine mature, qui ont été délétés par la digestion BamHI.

Un recombinant, dont la structure a été vérifiée par séquençage, a été retenu : pTG1999.

10

B. Microinjection du pTG1999 dans les oeufs de souris

On sait que la présence de séquences du vecteur (pBR322, pUC8, pUC18) peut avoir un effet inhibiteur sur l'expression d'un transgène dans l'animal. Pour débarrasser la construction Ig-$\alpha$AT du pTG1999 des séquences du vecteur, on digère le plasmide par SalI et XmnI (figure 8). Le fragment recherché, de 4,0 kb, est séparé des 2 autres fragments (de 0,8 et 1,8 kb) par centrifugation en gradient de sucrose. Le pool des fractions contenant le 4,0 kb est ensuite précipité à l'éthanol, redissous dans 200 $\mu$l de tampon TE, extrait au phénol-chloroforme, reprécipité à l'éthanol et redissous dans 10 $\mu$l de tampon TE. La solution d'ADN finale est analysée par électrophorèse sur gel d'agarose pour vérifier l'absence de traces de fragments de vecteur contaminant.

Cette préparation d'ADN à 40 ng/ml est injectée dans les oeufs fertilisés de souris. On injecte environ 500 copies par oeuf. Au total, on a injecté 1014 oeufs qui ont été réimplantés dans 72 souris femelles. On a obtenu 158 souriceaux.

On analyse l'ADN de chacun des souriceaux, sur une préparation d'ADN extrait à partir des queues des souriceaux, par hybridation avec une sonde radioactive dérivée du pTG1990 par "nick translation". Un seul souriceau donne un signal positif indiquant la présence du gène, il a été numéroté S.TG1999/1.

Une deuxième expérience a été réalisée dans les mêmes conditions ; on a injecté 706 oeufs ; ils ont été réimplantés dans 32 souris femelles ; on a obtenu 87 souriceaux vivants. Parmi ceux-ci, 7 ont intégré le transgène : S.TG1999/2 à 8.

La souris S.TG1999/1 est une femelle. Elle a été accouplée avec un mâle et on a étudié sa descendance pour vérifier si le transgène était stable d'une génération à l'autre. Parmi 19 souriceaux obtenus en 3 portées, 5 portaient le transgène. Il est donc plus probable que la souris S.TG1999/1 était une "mosaïque" plutôt qu'une souris homogène dont chaque cellule avait intégré le gène.

C. Mesure de l'$\alpha$-antitrypsine dans le sang de la souris transgénique S.TG1999/1

La quantité d'$\alpha$-antitrypsine présence dans le plasma de la souris transgénique a été déterminée par un test Elisa (décrit dans le paragraphe Méthodes).

Les résultats sont présentés dans le tableau I.

On détecte une quantité significative d'$\alpha$-AT : de 2 à 8 $\mu$g/ml. Le modèle de mise du gène étranger sous la dépendance des éléments de contrôle de transcription et traduction des immunoglobulines est donc bien fonctionnel dans la souris.

D. Production d'$\alpha_1$-antitrypsine par des hybridomes dérivés de la souris S.TG1999/1

La souris S.TG1999/1 a été stimulée avec de l'adjuvant de Freund et sacrifiée une semaine plus tard. A ce moment on détectait 15 $\mu$g/ml d'$\alpha$-AT dans le sang.

Les lymphocytes de la rate ont été préparés et fusionnés avec les cellules de myélome P3.X63Ag8.653.

48 clones ont été obtenus et les surnageants des cultures analysées par Elisa.

Les 2 clones meilleurs producteurs, A2 et A5, produisent 3 ng/ml.

Après sous-clonage on a obtenu une production de 10-15 ng/ml après 5 jours de culture.

Exemple 2 Induction de tumeurs dans des souris transgéniques avec des vecteurs comportant le grand antigène T de SV40 et le bloc d'expression de l'$\alpha_1$-antitrypsine humaine.

A. Construction du vecteur

On récupère les éléments du SV40, la totalité du gène du grand antigène T et le signal de polyadénylation, à partir d'un plasmide qui a été construit pour exprimer la glycoprotéine de la rage, pTG173 (brevet français 83.15716) mais tout autre plasmide portant les mêmes séquences pourrait être utilisé.

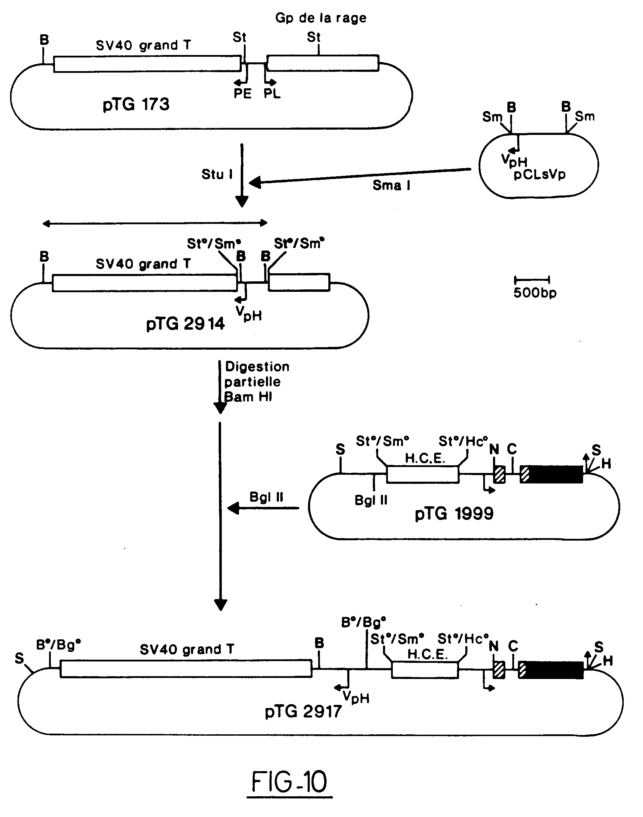

Le plasmide pTG173 (figure 10) comprend, entre les nucléotides 2533 et 346, la totalité du grand antigène T de SV40 avec son signal de polyadénylation. Il peut être récupéré sous forme d'un fragment StuI-BamHI de 2,65 kb mais la coupure par StuI délète la région promoteur précoce jusqu'à l'ATG initiateur.

Pour introduire un promoteur d'immunoglobuline en amont du gène T de SV40, on a digéré le plasmide pTG173 avec StuI, qui coupe le plasmide une deuxième fois dans le gène de la protéine rabique (figure 10).

Pour récupérer le promoteur de la chaîne lourde de l'immunoglobuline de souris, on utilise un plasmide pCLSvp, dérivé de pUC18 qui contient 2 polylinkers et un insert de 40O pb dérivé de l'hybridome NP-186-2 décrit par Bothwell et al. (1981).

Un fragment SmaI-SmaI est choisi pour être introduit dans le grand fragment StuI-StuI du pTG173 (figure 10), par ligation d'extrémités à bouts francs. Les transformants obtenus sont analysés pour déterminer l'orientation de l'insert Ig et un candidat est retenu : pTG2914.

La dernière étape de cette construction est l'insertion des séquences promoteur Ig-gène T de SV40, sous forme d'un fragment BamHI, dans le site BglII du pTG1999 (décrit dans l'exemple 1).

Le promoteur de la chaîne lourde d'Ig se retrouve ainsi sous la séquence "enhancer" de la même Ig et la transcription qu'il contrôle se fait en sens opposé de celle du gène α-AT (figure 10).

Cette étape nécessite une digestion partielle par BamHI, dans des conditions bien contrôlées, parce qu'il existe aussi un site BamHI entre l'antigène T et le promoteur Ig.

Le fragment d'ADN partiellement digéré par BamHI est ensuite inséré dans le pTG1999 ouvert par BglII et traité à la phosphatase.

Les transformants sont analysés pour l'orientation respective de leurs inserts et pour vérifier qu'il n'y a pas eu de réarrangement dans l'insert de 3.5 kb pendant le clonage. Un candidat a été retenu : pTG2917.

Comme dans l'exemple précédent, cette construction a été débarrassées des séquences du vecteur, par digestion cette fois avec l'enzyme FnuDII qui coupe plusieurs fois dans le vecteur mais pas dans le bloc d'expression. Le fragment FnuDII de 2,7 kb a été purifié en gradient de sucrose et préparé pour la microinjection, comme décrit dans l'exemple 1.

B. Injection du fragment FnuDII de 7,2 kb du pTG2917 dans les oeufs de souris

On injecte environ 500 copies par oeuf ; 1166 oeufs ont été injectés et réimplantés dans 104 femelles ; on a obtenu 78 souriceaux vivants ; parmi ceux-ci, 11 ont intégré le transgène.

C. Détermination du taux d'α-antitrypsine dans le plasma des souris transgéniques S.TG-2917

Les taux d'α-AT mesurés dans les plasmas des souris transgéniques sont présentés dans le tableau I (9 souris - 2 sont mortes pendant les manipulations).

Les souris qui ont survécu assez longtemps ont été étudiées une deuxième fois. Les autres sont mortes soit au cours des prises de sang par ponction cardiaque, soit à cause de la croissance de leurs tumeurs.

Dans les souris S.TG2917/2, 4, 7 et 9, l'augmentation du taux d'α-AT mesuré à la deuxième prise de sang est probablement dûe au développement des tumeurs.

D. Pathologie des souris transgéniques et formation des tumeurs

Jusqu'au moment du développement des tumeurs, les souris transgéniques ne se distinguent pas fort des souris normales de la même portée. Cependant certains animaux présentent une morphologie particulière de la tête qui est plus petite et plus ronde et cette caractéristique est transmissible à la descendance. De plus certains animaux présentent des troubles neurologiques et moteurs. La cause précise de ces troubles n'est pas connue.

Le développement des tumeurs se produit entre 10 et 18 semaines après la naissance. Dans la plupart des cas on observe une hypertrophie des ganglions lymphoïdes et du thymus. On observe parfois de grosses masses tumorales dérivées des ganglions lymphoïdes mésentériques ou brachiaux. On peut observer des hyper- ou des hypo-trophies de la rate et parfois un élargissement des glandes salivaires. On ne trouve pas de tumeurs dans le cerveau alors qu'on en décrit pour des tumeurs induites par l'antigène T du SV40 sous le contrôle du promoteur précoce de SV40.

Tableau I — Détermination du taux d'$\alpha_1$-AT dans le plasma des souris transgéniques portant le fragment SalI du pTG2917.

| Souris | Age au moment de la mort | µg/ml d'$\alpha$-AT dans le plasma 11.02.1987 | 2ème prise | cause de la mort | remarques |
|---|---|---|---|---|---|
| S.TG2917/1 | 13 | 0,32 | | défaillance cardiaque pendant prise de sang | descendance |
| S.TG2917/2 | 18 | 0,92 | 2,4 18.3.87 | sacrifiée - nombreuses tumeurs lymphoïdes | |
| S.TG2917/3 | 11 | 0,8 | | occlusion intestinale causée par tumeurs des ganglions mésentériques | |
| S.TG2917/4 | 18 | 1 | 2 25.3.87 | sacrifiée - nombreuses tumeurs | descendance |
| S.TG2917/5 | 13 | 0,72 | | sacrifiée - nombreuses tumeurs | |
| S.TG2917/6 | vivante | 0 | | - | faux transgénique (dé montré plus tard |
| S.TG2917/7 | 14 | 0,8 | 3,2 18.3.87 | sacrifiée - nombreuses tumeurs lymphoïdes | tête arrondie ; diffi cultés motrices ; descendance |
| S.TG2917/8 | 10 | 2,48 | | sacrifiée - nombreuses tumeurs lymphoïdes | |
| S.TG2917/9 | 14 | 0,6 | 3,8 18.3.87 | sacrifiée - nombreuses tumeurs lymphoïdes | tête arrondie ; diffi cultés motrices ; descendance |

E. Propagation des cellules tumorales in vivo

La propagation des cellules des tumeurs choisies des souris transgéniques peut s'effectuer par injection sous-cutanée ou intrapéritonéale de $10^6$ à $10^7$ cellules récupérées à partir d'une masse tumorale (par pression légère sur la masse cellulaire), en milieu Eagle + 10 % FC sérum. Les souris réceptrices sont des hybrides C57xSJL.

On a tenté la propagation des cellules tumorales des souris S.TG2917/1, 2917/4, 2917/7 et 2917/8. Seuls les extraits de STG2917/8 ont induit le développement de nouvelles tumeurs.

L'étude post-mortem de ces souris montre une hypertrophie de la rate, du thymus, de l'utérus. L'examen histologique de ces tissus montre une invasion par un grand nombre de cellules d'origine lymphoïde. Ces cellules sont plus petites que celles qu'on observe dans les tumeurs des souris transgéniques S.TG2926 (exemple suivant) ce qui indique un stade de développement ultérieur.

On a examiné le plasma de ces souris portant des tumeurs de 2ème génération : on y trouve de 0,5 à 1 $\mu$g/ml d'$\alpha$-AT, selon la souris.

F. Propagation des cellules tumorales in vitro

On prélève un fragment de tissu tumoral, après la mort de la souris, et on le place dans 5 ml de milieu de culture Eagle + 10 % FC sérum + 50 $\mu$M mercaptoéthanol + 100 $\mu$M asparagine, et on injecte doucement le milieu dans la masse tumorale, pour dissocier les cellules. On récupère les cellules et on inocule entre $10^6$ et $10^7$ cellules par boîte de Pétri de diamètre de 2,5 cm. On incube les cultures à 37°C en atmosphère de 5 % $CO_2$, pendant 2 jours.

Le surnageant des cultures est ensuite mesuré en test Elisa pour déterminer la quantité d'$\alpha$-AT sécrétée.

On a obtenu 3 ng/ml.

Exemple 3 Induction de tumeurs dans les souris transgéniques par l'intermédiaire d'un vecteur comprtant le gène c-myc de souris et le bloc d'expression de l'$\alpha_1$-antitrypsine humaine

A. Construction du vecteur

Une construction semblable à celle du pTG2917 a été réalisée ; le gène T de SV40 a été remplacé par un fragment Xba-BamHI de 4,8 kb contenant les exons 2 et 3 du gène c-myc de souris.

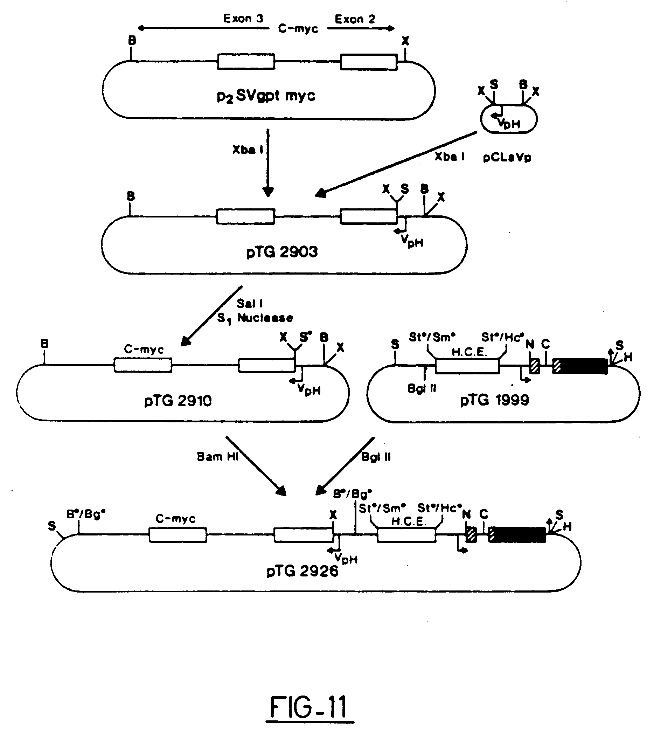

Ce fragment a été récupéré à partir du pSV2gpt/c-myc (voir figure 11). Ce plasmide (décrit par Bernard et al. 1983) contient un fragment XbaI-BamHI de 4,8 kb qui comprend les 2ème et 3ème exons du gène c-myc de souris (le 1er exon est non codant). Le promoteur de la chaîne lourde des Ig a été récupéré, dans ce cas-ci, sous forme de fragment XbaI, à partir du pCSLvp et introduit en amont du gène c-myc, dans le site XbaI du pSV2gpt. Un candidat a été retenu, pTG2903 ; l'orientation respective des inserts est notée dans la figure 11.

Il existe un site SalI entre le promoteur Ig et le gène c-myc. Pour faciliter les constructions ultérieures et récupérer l'ensemble comme un seul fragment de restriction, il est nécessaire de supprimer ce site. Pour cela on a ouvert le pTG2903 par SalI, digéré les extrémités simple brin par la nucléase $S_1$ et refermé le plasmide par la ligase T4.

On retient le clone pTG2910 ; l'analyse de sa carte de restriction confirme qu'il a perdu le site SalI mais que les site XbaI et SphI sont conservés, montrant que la digestion $S_1$ n'a pas été poussée trop loin.

Le fragment BamHI de 5,2 kb du pTG2910 contenant le promoteur de la chaîne lourde Ig et le gène c-myc a ensuite été introduit dans le site BglIII du pTG1999. On retient un transformant, pTG2926 dans lequel la transcription de c-myc s'opère en sens opposé de celle de l'$\alpha$-AT.

Pour préparer l'ADN à injecter dans les oeufs de souris, on élimine les séquences du vecteur, comme décrit plus haut, par digestion par SalI.

B. Injection du fragment SalI de 8,8 kb dans les oeufs de souris et obtention de souris transgéniques

On injecte environ 500 copies par oeuf ; on a injecté 1102 oeufs qui ont été réimplantés dans 50 souris femelles ; on a obtenir 69 souriceaux vivants dont 11 portent le transgène (S.TG2926/1 à 11).

Les résultats sont présentés dans le tableau II.

Comme dans l'exemple précédent, on mesure une quantité significative d'$\alpha$-AT dans le plasma.

Deux souris (S.TG2926/3 et 8) sont mortes avant d'avoir été examinées ; 5 souris sont mortes ou ont été sacrifiées entre 8 et 9 semaines après la naissance et présentaient de nombreuses tumeurs lymphoïdes ; 3 souris sont encore vivantes.

4 souris ont vécu assez longtemps pour être accouplées avec un mâle ; 2 seulement ont donné une descendance (S.TG2926/6 et 11).

14

## C. Pathologie des souris transgéniques et formation des tumeurs

Presque toutes les souris transgéniques de cette série sont plus petites que les souris normales de la même portée.

Les souris qui développent des tumeurs présentent en même temps un gonflement des ganglions lymphoïdes axiaux et brachiaux. Ces souris ont aussi des problèmes moteurs et finissent par ne plus bouger, tout en présentant des palpitations constantes.

Tableau II

| Détermination du taux d'$\alpha_1$-AT dans le plasma des souris transgéniques obtenues après injection du fragment SalI du pTG2926. | | | | | |
|---|---|---|---|---|---|
| Souris | Age au moment de la mort (semaines) | $\mu$g/ml d'$\alpha$-AT dans le plasma | | cause de la mort | remarques |
| | | 1ère prise | 2ème prise | | |
| S.TG2926/1 | encore vivante | 0,04 10.3.87 | 0 30.4.87 | - | descendance |
| S.TG2926/2 | 9 | 1,8 (le jour du sacrifice) | | sacrifiée - nombreuses tumeurs lymphoïdes | |
| S.TG2926/3 | 4 | NT | | pas examiné | |
| S.TG2926/4 | 8 | NT | | sacrifiée - nombreuses tumeurs | |
| S.TG2926/5 | 12 | NT | | morte de ses tumeurs | |
| S.TG2926/6 | 12 | 20 (le jour de sacrifice) | | sacrifiée - nombreuses tumeurs | descendance |
| S.TG2926/7 | encore vivante | 0,032 10.3.87 | 0 30.4.87 | - | |
| S.TG2926/8 | ? | NT | | - | |
| S.TG2926/9 | 8 | 1,13 (le jour de sacrifice) | | sacrifiée - nombreuses tumeurs lymphoïdes | |
| S.TG2926/10 | 8 | 2 (le jour de sacrifice) | | sacrifiée - nombreuses tumeurs lymphoïdes | |
| S.TG2926/11 | encore vivante | | 0,8 30.4.87 | - | descendance |

Le moment du développement de la tumeur et de la mort de l'animal varie d'une souris à l'autre. A l'examen post-mortem, elles présentent toutes une hypertrophie des ganglions lymphoïdes, du thymus et de la rate, et parfois d'importantes tumeurs dérivées des ganglions mésentériques ou brachiaux.

L'examen histologique de ces tumeurs montre une importante infiltration par des cellules d'origine lymphoïde, B ou T.

3 souris (S.TG2926/1, 7 et 11) sont encore vivantes 18 semaines après leur naissance et ne semblent pas affectées par la présence du transgène.

## D. Propagation des cellules tumorales in vivo

Les cellules sont récupérées à partir des tumeurs des ganglions brachiaux ou du thymus, comme décrit dans l'exemple précédent, et injectées par voie sous-cutanée ou intrapéritonéale, à une dose de $10^6$-$10^7$ cellules par souris (C57xSJL).

Toutes les souris injectées tombent malades dans les 2 semaines qui suivent l'injection, avec des symptômes semblables à ceux des souris transgéniques de première génération.

On mesure un taux d'$\alpha$-AT de 1 à 2 $\mu$g/ml dans le plasma.

Les cellules tumorales peuvent être récupérées et réinjectées sur de nouvelles souris : celles-ci développent les mêmes tumeurs et produisent de 1 à 2 $\mu$g/ml d'$\alpha$-AT dans le plasma.

L'examen histologique des souris réinjectées montre que les tumeurs ont la même morphologie et la même localisation que dans les premières souris transgéniques. On constate que les tumeurs induites par le pTG2926 ont un caractère très agressif et invasif.

On a tenté d'obtenir des tumeurs d'ascites d'Ehrlich, par injection intrapéritonéale des cellules tumorales dans des souris préalablement traitées au pristan. Les souris n'ont pas développé de tumeurs d'ascites mais ont présenté une hypertrophie des ganglions lymphatiques, surtout mésentériques, et la formation de tumeurs. Cette expérience montre donc la nécessité, pour la multiplication de ces cellules transformées, d'un environnement physiologique typique de tissu lymphoïde.

E. Propagation des cellules tumorales in vitro

Les cellules tumorales ont été mises en culture, comme dans l'exemple 2.

Le surnageant des cultures a été mesuré en test Elisa ; on obtient de 50 à 600 ng d'$\alpha$-AT/ml.

Il est donc possible d'obtenir des lignées cellulaires permanentes qui expriment et sécrètent l'$\alpha$-AT.

Les conditions de culture et de production peuvent encore être optimisées.

F. Etude de la descendance des souris transgéniques S.TG2926

Quelques souris ont donné une descendance (voir tableau II) ; la souris S.TG2926/6 a donné 15 souriceaux dont 8 portaient le transgène ; la S.TG2926/11 a donné 13 souriceaux dont 6 portaient le transgène.

La descendance des souris S.TG2926/1 et 7 ne portait pas le transgène.

Dépôt de souches représentatives de l'invention

Les souches suivantes ont été déposées à la Collection Nationale de Cultures de Microorganismes (25 Rue du Dr. Roux, Paris), le 15 juin 1987 :

5K/pTG1999 sous le n° I668

5K/pTG2917 sous le n° I669

5K/pTG2926 sous le n° I670.

REFERENCES

1) Palmiter, R.D. & Brinster, R.L. Ann. Rev. Genetics 20, 465-499 (1986).

2) Swift, G.H., Hammer, R.E., Mac Donald, R.J. & Brinster, R.L. Cell 38, 639-646 (1984).

3) Adams, J.M., Harris, A.W., Pinkert, C.A., Corcoran, L.M. & Alexander, W.S. Nature 318, 533-538 (1985).

4) Quaife, C.J., Pinkert, C.A., Ornitz, P.M., Palmiter, R.D. & Brinster, R.L. Cell, 1023-1034 (1987).

5) Rüther, U., Garber, Ch., Komitowski, Müller, R. & Wagner, E. F. Nature 325, 412-416 (1987).

6) Palmiter, R.D., Chen, H.Y., Messing, A. & Brinster, R.L. Nature 316, 457-460 (1985).

7) Babinet, C., Morello, D., Rougeot, C. & Rouyre, S. Eur. J. Immunol. 16, 1033-1035 (1986).

8) Babinet, C., Farza, H., Morello, D., Hadchouel, M. & Pourcel, C. Science 230, 1160-1163 (1985).

9) Sifers, R.N., Carlson, J.A., Clift, S.M., Demayo, F.J., Ballock, D.W. & Woo, S.L.C. Nucl. Acids Res. 15, 1459-1475 (1987).

10) Overbeek, P.A., Chepelinsky, A., Khillan, J.S., Piatigorsky, J. & Westphal, H. Proc. Natl. Acad. Sci. USA 82, 7815-7819 (1985).

11) Kelley, D.E., Coleclough, C. & Perry, R.P. Cell 29, 681-689 (1982).

12) Church, G., Ephrussi, A., Gilbert, W. & Tonegawa, S. Science 227, 134-140 (1985).

13) Bothwell, A.L.M., Paskind, M., Reth, M., Imamiski-Kari, T., Rajewsky, K. & Baltimore, D. Cell 24, 625-637 (1981).

14) Bernard, O., Cory, S., Gerondakis, S., Webb, E. & Adams, J.M. EMBO J. 2, 2375-2383 (1983).

EP 0 298 807 B1

**Revendications**

1. Procédé de préparation d'une lignée cellulaire stable produisant une protéine déterminée, caractérisé en ce que :
   a) - on prépare un vecteur comportant
      - une séquence oncogène ainsi que les éléments assurant son expression dans une cellule eucaryote supérieure,
      - un bloc d'expression qui comprend au moins la séquence codant pour ladite protéine ainsi que les éléments assurant son expression dans ladite cellule eucaryote supérieure tumorale ; ladite protéine étant différente de celle exprimée par ladite séquence oncogène,
   b) - on introduit ce vecteur dans un ou plusieurs oeufs d'un animal et on réimplante le ou les oeufs obtenus ;
   c) - on sélectionne les descendants ayant intégré l'oncogène et qui développent des tumeurs ;
   d) - on prélève et on cultive sur un milieu approprié les cellules tumorales ainsi obtenues et qui produisent la protéine déterminée afin d'obtenir une lignée cellulaire.

2. Procédé selon la revendication 1, caractérisé en ce qu'avant d'être cultivées à l'étape d), les cellules tumorales prélevées sont réinjectées dans un autre animal pour induire le développement de nouvelles tumeurs dont les cellules seront cultivées pour obtenir une lignée cellulaire.

3. Procédé selon l'une des revendications 1 et 2, caractérisé en ce que les cellules tumorales prélevées et cultivées sont celles d'un animal d'une génération suivante développant également des tumeurs.

4. Procédé selon l'une des revendications 1 à 3, caractérisé en ce que l'oncogène est choisi pour pouvoir s'exprimer dans l'animal traité.

5. Procédé selon la revendication 4, caractérisé en ce que l'oncogène est choisi parmi : c-myc, c-ras, c-fos et l'antigène T de SV40.

6. Procédé selon la revendication 1, caractérisé en ce que :
   a) - on prépare un vecteur comportant
      - un bloc d'expression qui comprend au moins la séquence codant pour ladite protéine ainsi que les éléments assurant son expression dans une cellule eucaryote supérieure ;
   b) - on introduit ce vecteur dans un ou plusieurs oeufs d'un animal et on réimplante le ou les oeufs obtenus ;
   c) - on sélectionne les descendants ayant intégré la séquence codant pour ladite protéine ;
   d) - on fusionne les lymphocytes de la rate de ces animaux avec des cellules de myélome et on sélectionne des clones d'hybridome produisant la protéine.

7. Procédé selon l'une des revendications 1 à 6, caractérisé en ce que le vecteur est pratiquement dépourvu d'ADN bactérien.

8. Procédé selon l'une des revendications 1 à 7, caractérisé en ce que le vecteur est un fragment d'ADN linéaire.

9. Procédé selon l'une des revendications 1 à 8, caractérisé en ce que les éléments assurant l'expression de l'oncogène et ceux assurant l'expression de la protéine déterminée sont spécifiques d'un tissu ou d'un type de cellule.

10. Procédé selon l'une des revendications 1 à 9, caractérisé en ce que les éléments assurant l'expression comprennent un promoteur et un activateur de transcription.

11. Procédé selon l'une des revendications 1 à 10, caractérisé en ce que le gène codant pour ladite protéine est associé à un gène d'amplification et en ce que l'on amplifie le nombre de copies du gène codant pour ladite protéine en utilisant le gène d'amplification dans les cellules obtenues.

12. Procédé selon la revendication 11, caractérisé en ce que le gène d'amplification est choisi parmi le gène de la dihydrofolate réductase ou de l'adénine deaminase.

17

EP 0 298 807 B1

**13.** Procédé selon l'une des revendications 1 à 12, caractérisé en ce que pour l'expression de l'oncogène et de la protéine dans une cellule lymphoïde, les éléments assurant l'expression sont les éléments régulateurs de l'immunoglobuline.

**14.** Procédé selon la revendication 13, caractérisé en ce que le ou les promoteurs sont choisi parmi :
- . P.Ig (LC) qui représente le promoteur de la chaîne légère des immunoglobulines, et
- . P.Ig (HC) qui représente le promoteur de la chaîne lourde des immunoglobulines,
et l'activateur de transcription est :
- . (enh).Ig qui représente le "enhancer" de la chaîne lourde des immunoglobulines.

**15.** Procédé selon l'une des revendications 1 à 12, caractérisé en ce que pour l'expression de l'oncogène et de la protéine dans les cellules hépatiques, les éléments assurant l'expression sont des éléments régulateurs de la synthèse d'une protéine hépatique ou d'un virus hépatique.

**16.** Procédé selon la revendication 15, caractérisé en ce que le promoteur est choisi parmi les promoteurs de l'alpha-antitrypsine, de l'albumine et l'activateur de transcription est celui du virus de l'hépatite B.

**17.** Procédé selon l'une des revendications 1 à 16, caractérisé en ce que l'animal est choisi parmi les mammifères, les oiseaux, les reptiles et les poissons.

**18.** Procédé selon l'une des revendications 1 à 17, caractérisé en ce que la protéine déterminée est une protéine d'intérêt thérapeutique choisie parmi les protéines humaines ou animales et les antigènes viraux ou d'autre origine.

**19.** Procédé selon la revendication 18, caractérisé en ce que la protéine déterminée est une protéine humaine d'origine hépatique et en ce que la lignée cellulaire obtenue est une lignée de tumeur hépatique.

**20.** Procédé selon la revendication 18, caractérisé en ce que la protéine déterminée est choisie parmi :
- . l'alpha-antitrypsine,
- . les facteurs de coagulation du sang,
- . leurs variants ou analogues ayant ou non sensiblement la même activité thérapeutique.

**21.** Procédé selon la revendication 20, caractérisé en ce que la protéine déterminée est choisie parmi le facteur VIII et le facteur IX.

**22.** Animal non-humain transgénique obtenu par la mise en oeuvre du procédé selon l'une des revendications 1 à 21 pour la production d'une protéine d'activité thérapeutique.

**23.** Animal selon la revendication 22, caractérisé en ce qu'il s'agit d'une souris.

**24.** Cellule d'un animal transgénique selon l'une des revendications 22 ou 23, caractérisée en ce qu'elle exprime l'oncogène et la protéine déterminée.

**25.** Cellule selon la revendication 24, caractérisée en ce qu'il s'agit d'une cellule lymphoïde ou d'une cellule hépatique.

**26.** Tumeurs résultant de la transformation des cellules selon l'une des revendications 24 ou 25.

**27.** Lignées cellulaires pouvant être obtenues par le procédé selon l'une des revendications 1 à 21, ou dérivant des tumeurs selon la revendication 26.

**28.** Procédé de préparation d'une protéine déterminée à partir de cellules eucaryotes supérieures, caractérisé en ce qu'on utilise les lignées cellulaires selon la revendication 27 et on récupère la protéine obtenue.

18

**Claims**

1. Process for preparing a stable cell line producing a specific protein, characterized in that:
   a) - a vector is prepared comprising
      - an oncogenic sequence as well as the elements ensuring its expression in a higher eucaryotic cell,
      - an expression block which comprises at least the sequence encoding the said protein as well as the elements ensuring its expression in the said higher eucaryotic tumour cell; the said protein being different from that expressed by the said oncogenic sequence;
   b) - this vector is introduced into one or more eggs of an animal and the egg(s) obtained is (are) reimplanted;
   c) - the progeny having integrated the oncogene and which develop tumours are selected;
   d) - the tumour cells thus obtained and which produce the specific protein are collected and cultured on an appropriate medium so as to obtain a cell line.

2. Process according to Claim 1, characterized in that before being cultured in step d), the tumour cells collected are reinjected into another animal in order to induce the development of new tumours whose cells will be cultured in order to obtain a cell line.

3. Process according to either of Claims 1 and 2, characterized in that the tumour cells collected and cultured are those of an animal of a following generation also developing tumours.

4. Process according to one of Claims 1 to 3, characterized in that the oncogene is chosen so as to be expressible in the treated animal.

5. Process according to Claim 4, characterized in that the oncogene is chosen from: c-myc, c-ras, c-fos and SV40 T antigen.

6. Process according to Claim 1, characterized in that:
   a) - a vector is prepared comprising
      - an expression block which comprises at least the sequence encoding the said protein as well as the elements ensuring its expression in a higher eucaryotic cell;
   b) - this vector is introduced into one or more eggs of an animal and the egg(s) obtained is (are) reimplanted;
   c) - the progeny having integrated the sequence encoding the said protein are selected;
   d) - the lymphocytes of the spleen of these animals are fused with myeloma cells and hybridoma clones producing the protein are selected.

7. Process according to one of Claims 1 to 6, characterized in that the vector is practically devoid of bacterial DNA.

8. Process according to one of Claims 1 to 7, characterized in that the vector is a linear DNA fragment.

9. Process according to one of Claims 1 to 8, characterized in that the elements ensuring the expression of the oncogene and thus ensuring the expression of the specific protein are specific for a tissue or for a cell type.

10. Process according to one of Claims 1 to 9, characterized in that the elements ensuring the expression comprise a promoter and a transcription activator.

11. Process according to one of Claims 1 to 10, characterized in that the gene encoding the said protein is associated with an amplification gene and in that the copy number of the gene encoding the said protein is amplified using the amplification gene in the cells obtained.

12. Process according to Claim 11, characterized in that the amplification gene is chosen from the dihydrofolate reductase or adenine deaminase gene.

EP 0 298 807 B1

**13.** Process according to one of Claims 1 to 12, characterized in that for the expression of the oncogene and the protein in a lymphoid cell, the elements ensuring the expression are the immunoglobulin regulator elements.

**14.** Process according to Claim 13, characterized in that the promoter(s) is (are) chosen from:
- . P.Ig (LC) which represents the immunoglobulin light chain promoter, and
- . P.Ig (HC) represents the immunoglobulin heavy chain promoter,

and the transcription activator is:
- . (enh).Ig which represents the immunoglobulin heavy chain "enhancer".

**15.** Process according to one of Claims 1 to 12, characterized in that for the expression of the oncogene and the protein in hepatic cells, the elements ensuring the expression are regulator elements for the synthesis of a hepatic protein or of a hepatic virus.

**16.** Process according to Claim 15, characterized in that the promoter is chosen from the alpha-antitrypsin and albumin promoters and the transcription activator is that of the hepatitis B virus.

**17.** Process according to one of Claims 1 to 16, characterized in that the animal is chosen from mammals, birds, reptiles and fish.

**18.** Process according to one of Claims 1 to 17, characterized in that the specific protein is a protein of therapeutic interest chosen from human or animal proteins and viral antigens or antigens of another origin.

**19.** Process according to Claim 18, characterized in that the specific protein is a human protein of hepatic origin and in that the cell line obtained is a hepatic tumour line.

**20.** Process according to Claim 18, characterized in that the specific protein is chosen from
- . alpha-antitrypsin,
- . blood coagulation factors,
- . their variant or analogues having or otherwise substantially the same therapeutic activity.

**21.** Process according to Claim 20, characterized in that the specific protein is chosen from factor VIII and factor IX.

**22.** Transgenic non-human animal obtained using the process according to one of Claims 1 to 21 for the production of a protein with therapeutic activity.

**23.** Animal according to Claim 22, characterized in that it is a mouse.

**24.** Cell of a transgenic animal according to either of Claims 22 and 23, characterized in that it expresses the oncogene and the specific protein.

**25.** Cell according to Claim 24, characterized in that it is a lymphoid cell or a hepatic cell.

**26.** Tumours resulting from the transformation of the cells according to either of Claims 24 and 25.

**27.** Cell lines capable of being obtained by the process according to one of Claims 1 to 21, or derived from the tumours according to Claim 26.

**28.** Process for preparing a specific protein from higher eucaryotic cells, characterized in that the cell lines according to Claim 27 are used and the protein obtained is recovered.

**Patentansprüche**

**1.** Verfahren zur Herstellung einer stabilen Zellinie, die ein vorgegebenes Protein bildet, dadurch gekennzeichnet, daß man
   a) einen Vektor herstellt, der umfaßt

20

EP 0 298 807 B1

- eine onkogene Sequenz sowie die Elemente, die ihre Expression in einer höheren Eukaryonten-Zelle gewährleisten,
- einen Expressions-Block, der mindestens die für das genannte Protein codierende Sequenz sowie die ihre Expression in einer höheren Eukaryonten-Tumorzelle gewährleistenden Elementen umfaßt, wobei das genannte Protein von demjenigen verschieden ist, das durch die genannte onkogene Sequenz exprimiert wird,

b) diesen Vektor in ein oder mehrere Eier eines Tieres einführt und das (die) erhaltene(n) Ei(Eier) wieder implantiert;

c) die Deszendenten, die das Onkogen integriert haben und Tumore entwickeln, selektioniert; und

d) die so erhaltenen Tumorzellen, die das vorgegebene Protein bilden, entnimmt und auf einem geeigneten Medium kultiviert (züchtet) zur Bildung einer Zellinie.

2. Verfahren nach Anspruch 1, dadurch gekennzeichnet, daß die entnommenen Tumorzellen vor der Kultivierung (Züchtung) in der Stufe (d) wieder in ein anderes Tier injiziert werden, um die Entwicklung von neuen Tumoren zu induzieren, deren Zellen kultiviert werden zur Bildung einer Zellinie.

3. Verfahren nach einem der Ansprüche 1 und 2, dadurch gekennzeichnet, daß die entnommenen und kultivierten (gezüchteten) Tumorzellen Zellen eines Tieres einer Nachfolgegeneration sind, die ebenfalls Tumore entwickeln.

4. Verfahren nach einem der Ansprüche 1 bis 3, dadurch gekennzeichnet, daß das Onkogen so ausgewählt wird, daß es sich in dem behandelten Tier exprimieren kann.

5. Verfahren nach Anspruch 4, dadurch gekennzeichnet, daß das Onkogen ausgewählt wird aus c-myc, c-ras, c-fos und dem Antigen T von SV40.

6. Verfahren nch Anspruch 1, dadurch gekennzeichnet, daß man

a) einen Vektor herstellt, der umfaßt
- einen Expression-Block, der mindestens die für das genannte Protein codierende Sequenz sowie die Elemente, die ihre Expression in einer höheren Eukaryonten-Zelle gewährleisten, umfaßt;

b) diesen Vektor in ein oder mehrere Eier eines Tieres einführt und das (die) erhaltene(n) Ei(Eier) wieder implantiert;

c) die Deszendenten, welche die für das genannte Protein codierende Sequenz integriert haben, selektioniert; und

d) die Lymphozyten der Milz dieser Tiere mit Myelom-Zellen fusioniert und Hybridom-Klone, die das Protein bilden, selektioniert.

7. Verfahren nach einem der Ansprüche 1 bis 6, dadurch gekennzeichnet, daß der Vektor praktisch frei von Bakterien-DNA ist.

8. Verfahren nach einem der Ansprüche 1 bis 7, dadurch gekennzeichnet, daß der Vektor ein lineares DNA-Fragment ist.

9. Verfahren nach einem der Ansprüche 1 bis 8, dadurch gekennzeichnet, daß die Elemente, welche die Expression des Onkogens gewährleisten, und diejenigen, welche die Expression des vorgegebenen Proteins gewährleisten, spezifisch sind für ein Gewebe oder für einen Zell-Typ.

10. Verfahren nach einem der Ansprüche 1 bis 9, dadurch gekennzeichnet, daß die Elemente, welche die Expression gewährleisten, einen Promotor und einen Transkriptions-Aktivator umfassen.

11. Verfahren nach einem der Ansprüche 1 bis 10, dadurch gekennzeichnet, daß das für das genannte Protein codierende Gen mit einem Amplifikations-Gen assoziiert ist und daß man die Anzahl der Kopien des Gens, das für das genannte Protein codiert, amplifiziert durch Verwendung des Amplifikations-Gens in den erhaltenen Zellen.

12. Verfahren nach Anspruch 11, dadurch gekennzeichnet, daß das Amplifikations-Gen ausgewählt wird unter dem Gen der Dihydrofolat-Reduktase oder der Adenin-Deaminase.

21

13. Verfahren nach einem der Ansprüche 1 bis 12, dadurch gekennzeichnet, daß für die Expression des Onkogens und des Proteins in einer Lymphoid-Zelle die Elemente, welche die Expression gewährleisten, Regulator-Elemente des Immunglobulins sind.

14. Verfahren nach Anspruch 13, dadurch gekennzeichnet, daß der oder die Promotoren ausgewählt werden aus:

. P.Ig (LC), das den Promotor für die leichte Kette der Immunglobuline darstellt, und
. P.Ig (LH), das den Promotor für die schwere Kette der Immunglobuline darstellt, und
. daß es sich bei dem Transkriptions-Aktivator handelt um
. (enh).Ig, das den "Verstärker" für die schwere Kette der Immunglobuline darstellt.

15. Verfahren nach einem der Ansprüche 1 bis 12, dadurch gekennzeichnet, daß für die Expression des Onkogens und des Proteins in den Leberzellen die Elemente, welche die Expression gewährleisten, Regulator-Elemente für die Synthese eines Leber-Proteins oder eines Leber-Virus sind.

16. Verfahren nach Anspruch 15, dadurch gekennzeichnet, daß der Promotor ausgewählt wird unter den Promotoren von $\alpha$-Antitrypsin und Albumin und daß der Transkriptions-Aktivator derjenige des Hepatitis B-Virus ist.

17. Verfahren nach einem der Ansprüche 1 bis 16, dadurch gekennzeichnet, daß das Tier ausgewählt wird unter den Säugetieren, den Vögeln, den Reptilien und den Fischen.

18. Verfahren nach einem der Ansprüche 1 bis 17, dadurch gekennzeichnet, daß das vorgegebene Protein ein Protein von therapeutischem Interesse (Vorteil) ist, das ausgewählt wird unter den Human- oder Tier-Proteinen und den Virus-Antigenen oder Antigenen eines anderen Ursprungs.

19. Verfahren nach Anspruch 18, dadurch gekennzeichnet, daß das vorgegebene Protein ein Human-Protein ist, das aus der Leber stammt, und daß die erhaltene Zellinie eine Lebertumor-Linie ist.

20. Verfahren nach Anspruch 18, dadurch gekennzeichnet, daß das vorgegebene Protein ausgewählt wird unter

. $\alpha$-Antitrypsin,
. den Blut-Koagulationsfaktoren,
. ihren Varianten oder Analoga, die im wesentlichen die gleiche therapeutische Aktivität aufweisen oder nicht aufweisen.

21. Verfahren nach Anspruch 20, dadurch gekennzeichnet, daß das vorgegebene Protein ausgewählt wird unter dem Faktor VIII und dem Faktor IX.

22. Nicht-humanes transgenes Tier, das erhalten wird durch Anwendung des Verfahrens nach einem der Ansprüche 1 bis 21 zur Produktion eines Proteins mit therapeutischer Aktivität.

23. Tier nach Anspruch 22, dadurch gekennzeichnet, daß es sich dabei um eine Maus handelt.

24. Zelle eines transgenen Tiers nach einem der Ansprüche 22 oder 23, dadurch gekennzeichnet, daß sie das Onkogen und das vorgegebene Protein exprimiert.

25. Zelle nach Anspruch 24, dadurch gekennzeichnet, daß es sich dabei um eine Lymphoid-Zelle oder um eine Leberzelle handelt.

26. Tumore, die aus der Transformation der Zelle nach einem der Ansprüche 24 oder 25 resultieren.

27. Zellinien, die nach dem Verfahren nach einem der Ansprüche 1 bis 21 erhalten werden können oder aus Tumoren nach Anspruch 26 stammen.

28. Verfahren zur Herstellung eines vorgegebenen Proteins aus höheren Eukaryonten-Zellen, dadurch gekennzeichnet, daß man die Zellinien nach Anspruch 27 verwendet und das erhaltene Protein gewinnt (abtrennt).

FIG-1

Sequences de souris     Sequence d'homme

Sequence de Virus SV40

Sal I      H.C.E.      ATG      Sal I

$V_{Lk}$

$\alpha_1 AT_{arg}$

S.S.

SV40
Signal de polyadenylation

## FIG_2

EP 0 298 807 B1

FIG_3

EP 0 298 807 B1

```
                                              mRNA
    - 15                                        ⟷                    + 15
    T T T G C A G T G A G A T A T G A A A T G C A|T C A C A C C
    A A A C G T C A C T C T A T A C T T T|A C G T A G T G T G G
                                          Met   His   His   Thr

                                                      │Nsi I
                                                      │nuclease S₁
                                                      ▼

    T T T G C A G T G A G A T A T G A A A              T C A C A C C
    A A A C G T C A C T C T A T A C A T T              A G T G T G G


OligoTG616 5' C T C G A G G T T A A C A T C G A T G A A A T G C A 3'  ── Ligation
OligoTG617 3' G A G C T C C A A T T G T A G C T A C T T T A C G T 5'

                              │Hpa I                    ▼              Nsi I
                              ▼

    T T T G C A G T G A G A T A T G A A A C T C G A G G T T A A C A T C G A T G A A A T G C A T C A C A C C
    A A A C G T C A C T C T A T A C T T T G A G C T C C A A T T G T A G C T A C T T|T A C G T A G T G T G G
                                                                                  Met   His   His   Thr
```

## FIG.4

FIG. 5

FIG-6

3'non traduit

Bam        Eco RV      Stop    Pst

Arg

$\alpha_1$AT(Arg) fragment dans m13TG1920

Simple brin
mTG1920

T A A C T G C C T C T C G C T C C T C A A C C C C T C C C C T C C A T C C C T G G C C C C C T C C T G G

Stop codon

A G G T A G G G    C C G G G G G A G

C

site Sma I

Oligonucleotide
TG566

MUTAGENESE

site Sma I

Simple brin
mTG1923

T A A C T G C C T C T C G C T C C T C A A C C C C T C C C C T C C A T C C C G G G C C C C C T C C T G G

Stop codon

## FIG-7

FIG-8

Sequences de souris     Sequence d'homme

Sequence de Virus SV40

Sal I             ATG           Sal I

H.C.E.

$V_{Lk}$

S.S.

$\alpha_1$ATarg

SV40
Signal de polyadenylation

Adaptateur tg 738/739   Cla I   Bam HI

ATACACATTAT|CGA TTTCTGTTTCCAGGTGTTGACGGAGAA|GATCCCCAGGGA
TATGTGTAATAGC|TAAAGACAAAGGTCCACAACTGCCTCTTCTAG|GGGTCCCT

Cla I                          Bam HI°

Site d'épissage 3'

lyValAspGlyGluAspProGlnGly

Sequence signal       $\alpha_1$AT(Arg) mature

Clivage

FIG_9

FIG-10

FIG-11